# EUROPEAN PATENT APPLICATION

(11) **EP 4 707 780 A1**
(43) Date of publication of application: **11.03.2026**
(21) Application number: 24908211.6
(22) Date of filing: 21.12.2024
(51) Int. Cl.: G01N 21/17, G01N 29/24, G02B 26/10, A61B 5/00, A61B 8/00

(54) **LONG-STROKE VIBRATION-FREE LINEAR HIGH-SPEED RECIPROCATING SCANNING DEVICE**

(30) Priority: 22.12.2023 KR 20230189614; 08.08.2024 KR 20240106446; 13.12.2024 KR 20240186376; 20.12.2024 KR 20240193356
(71) Applicant: Ulsan National Institute of Science and Technology (UNIST), Eonyang-eup Ulju-gun Ulsan 44919 (KR)
(72) Inventor: YOUM, Jin Young, Ulju-gun, Ulsan 44919 (KR); YANG, Joon-Mo, Ulju-gun, Ulsan 44919 (KR)
(74) Representative: dompatent
(86) International application number: PCT/KR2024/020915
(87) International publication number: WO 2025/135929

(57) **Abstract**

Provided is a wide-range vibration-free high-speed linear reciprocating scanning device capable of solving mechanical vibration problems during transfer of a scanning head.

## Description

### [TECHNICAL FIELD]

The present disclosure relates to a device applicable not only to the field of photoacoustic imaging but also to general mechanical engineering, and more particularly to a photoacoustic scanner device that may be applied to small imaging devices such as handheld probes or endoscopic probes employing a single ultrasonic transducer-based mechanical scanning method, and to a scanning device that may be widely applied to various fields requiring mechanical reciprocating scanning in general mechanical engineering.

### [BACKGROUND ART]

Generally, a photoacoustic imaging device using a single ultrasonic transducer-based mechanical scanning method refers to a device that performs scanning by physically moving certain components to acquire a desired image over a specific range according to the principle of photoacoustic imaging.

A single ultrasonic transducer-based mechanical scanning method may implement a system much more economically than an electrical scanning method using an array transducer composed of multiple piezoelectric elements, and it may also provide significantly higher image resolution, and accordingly, various approaches, such as a method of steering only a laser beam using a galvanometer scanner, and methods of simultaneously steering both a laser beam and ultrasound by applying a MEMS scanner or a polygonal mirror, have been proposed. However, the scanning methods mentioned above are specialized for high-speed scanning, but in terms of the actual scanning range (stroke) in which an image may be acquired, they are generally very narrow, and typically 5 mm or less.

However, to apply photoacoustic imaging devices to actual clinical use, there is a requirement that the image scanning range has to reach a predetermined level or more, not just the scanning speed, and to satisfy this requirement, as in the examples mentioned above, simply steering only the laser beam or the ultrasonic beam in an angular manner has an inherent limitation in principle, making it impossible to cover the entire required scanning area.

Therefore, in this field, to satisfy the requirement for wide-range image scanning capability, a method, in which the related optical system or ultrasonic transducer is mounted on a stage and physically moved for scanning, has been applied, and most systems of this type that have been proposed thus far have been implemented using a structure that directly guides laser pulses generated from a light source to a scanning head, where the imaging is actually performed, through an optical fiber.

However, when mechanical scanning is performed over a section (stroke) wider than several millimeters while an optical fiber for light guidance is directly connected to the scanning head as described above, the bending of the optical fiber near the scanning head changes from moment to moment, and as a result, the intensity of laser pulses transmitted inside the optical fiber by the principle of total internal reflection becomes unstable, which in turn causes fluctuations in the amount of light delivered to the target tissue, and this problem becomes more serious as the scanning range increases and when spectral or functional imaging that requires the use of two or more wavelengths is performed, ultimately making it difficult to obtain accurate quantitative images.

Of course, when a single-mode optical fiber having an extremely fine core of 10 µm or less is used, the problem of light intensity nonuniformity mentioned above may be somewhat alleviated, but, considering the fundamental principle that the optical fiber itself transmits light based on total internal reflection, it is evident that geometrical shape changes will cause variations in transmission efficiency, and consequently, to reduce such problems, the scanning range has to ultimately be set to be very narrow, such as within several millimeters.

In addition, the bending problem of the optical fiber during a mechanical scanning process may cause the optical fiber to experience physical fatigue when a much thicker multimode optical fiber is used instead of a single-mode fiber to perform high-speed scanning over a range much wider than several millimeters, thereby resulting in fiber breakage, and elasticity generated as the curvature of the optical fiber changes, and temporal fluctuation of the elastic modulus, are applied as a kind of resistance when the scanning speed increases, and consequently, hamper an increase in scanning speed and lower the uniformity of the scanning speed.

The present disclosure provides a novel concept of a mechanical scanning device, and a photoacoustic scanner structure based thereon, which may solve not only the conventional problems of nonuniform light transmission caused by bending of a guiding optical fiber, mechanical resistance generated by the guiding optical fiber, and a narrow scanning range in existing photoacoustic scanner devices, but also a mechanical vibration problem that has occurred in all mechanical engineering fields when performing high-speed linear reciprocating scanning over a wide area of a target area.

### [DETAILED DESCRIPTION OF THE INVENTION]

### [TECHNICAL PROBLEM]

An embodiment disclosed in the present disclosure provides a wide-range vibration-free linear high-speed reciprocating scanning device that may solve mechanical vibration problems during high-speed reciprocating transfer of a scanning head for scanning operations, thereby providing a very high level of scanning uniformity over an entire target scanning range.

However, problems to be solved by the present disclosure are not limited to the problems mentioned above, and other problems that are not mentioned will be clearly understood by those skilled in the art from the following description.

### [TECHNICAL SOLUTION]

A photoacoustic scanner according to the present disclosure may acquire high-quality images with ensured uniformity of signal detection at high speed over any scanning area without any mechanical vibration, and an embodiment equipped with elements required for photoacoustic imaging will be described below.

A photoacoustic scanner according to the present disclosure for achieving the above-described technical problems may include a collimator that generates collimated light while being fastened to an optical fiber that delivers light from a light source, a scanning head that scans a target tissue based on the collimated light generated by the collimator, and a linear actuator that is connected to the scanning head to control a reciprocating movement of the scanning head.

Furthermore, the scanning head may include a prism that receives the collimated light from the collimator and changes a travel path of the collimated light, an illumination optical part that receives the collimated light from the prism and induces a photoacoustic signal by illuminating light to the target tissue according to a specific illumination pattern, an ultrasonic transducer that detects a photoacoustic wave induced by the illumination optical part, and a scanning head frame that fixes the prism, the illumination optical part, and the ultrasonic transducer.

Furthermore, the linear actuator may be connected to the scanning head frame.

Furthermore, the linear actuator may include a fixed part and a movable part that is connected to the scanning head while being reciprocated in the X-axis direction by a driving force provided from the fixed part.

Furthermore, a base frame that fixes the fixed part of the linear actuator and the collimator may be further included.

Furthermore, a stage that is disposed at a lower portion of the base frame and moves the base frame along the Y-axis direction may be further included.

Furthermore, the stage may include a support plate and a movable plate that is attached to the base frame while being moved along the +Y-axis direction and the -Y-axis direction by a driving force provided from the support plate.

Furthermore, a plurality of optical fibers, a plurality of collimators, and a plurality of light sources may be provided, the plurality of light sources may provide lights having different wavelengths, the plurality of optical fibers may be connected to the plurality of light sources, and the plurality of collimators may receive the plurality of lights from the plurality of light sources through the plurality of optical fibers to generate a plurality of collimated lights.

Furthermore, a reflective mirror that changes a travel path of collimated light from any one of the plurality of collimators, and a beam combiner that combines the collimated light from the any one collimator and the collimated light from another collimator to provide the combined light to the scanning head may be further included.

Furthermore, the plurality of collimators may include a first collimator that generates a first collimated light and a second collimator that generates a second collimated light.

Furthermore, a reflective mirror that changes a travel path of the second collimated light from the second collimator, and a beam combiner that combines the first collimated light from the first collimator and the second collimated light from the second collimator to provide the combined light to the scanning head may be further included.

Furthermore, the linear actuator may further include a first support part that is connected to the movable part of the linear actuator, and a second support part that is connected to the first support part and the scanning head, and in this case, the first support part may be further connected to the scanning head frame, and the second support part may be further connected to the illumination optical part. Furthermore, the first support part and the second support part may extend in crossing directions, and the first support part may extend in the Z-axis direction, and the second support part may extend in the X-axis direction. Furthermore, the first support part and the second support part may be formed integrally.

Furthermore, the linear actuator may further include a housing, in which the collimator and the linear actuator are disposed, and the collimator and the linear actuator may be attached to the housing.

Furthermore, the illumination optical part may include a single convex lens, and the ultrasonic transducer may be disposed at a central lower point of the single convex lens.

Furthermore, the scanning head may include a plane mirror that receives the collimated light from the collimator and changes a travel path of the collimated light, an illumination optical part that receives the collimated light from the plane mirror and induces a photoacoustic signal by illuminating light to the target tissue according to a specific illumination pattern, an ultrasonic transducer that detects a photoacoustic wave induced by the illumination optical part, and a scanning head frame that fixes the plane mirror, the illumination optical part, and the ultrasonic transducer, wherein the illumination optical part may include a single convex lens, and the ultrasonic transducer may be disposed at a central lower point of the single convex lens.

Furthermore, the ultrasonic transducer may be disposed in an interior of the illumination optical part.

Furthermore, the illumination optical part may be disposed in an interior of the ultrasonic transducer.

Furthermore, the ultrasonic transducer may be a ring transducer having a ring-shaped opening.

A wide-range vibration-free linear reciprocating scanning device for transferring a scanning head according to the present disclosure may include a first bevel gear that is rotated by a driving motor; a crank that is coupled to the first bevel gear and converts a rotational motion of the first bevel gear into a linear motion in association with a first link and a second link; a first link and a second link that are connected to the crank and are linearly moved in response to the linear motion converted by the crank; and a first slider and a second slider that are connected to the first link and the second link, respectively, and are linearly moved in response to the linear motions of the first link and the second link, and the scanning head is connected to the first slider and is linearly moved in response to the linear motion of the first slider.

Furthermore, the crank may include a central shaft that is coupled to the first bevel gear; two first connecting arms that are connected to opposite sides of the central shaft, respectively; two first shafts that are connected to the two first connecting arms, respectively, and to which the first link is coupled to be rotatable; two second connecting arms that are connected to the two first shafts, respectively; and two second shafts that are connected to the two second connecting arms, respectively, and to which the second link is coupled to be rotatable.

Furthermore, the central shaft may be connected to a center of the first connecting arm, the second connecting arm may be disposed symmetrically to the first connecting arm, a rotation center of the first connecting arm may be a center of the first connecting arm, and a rotation center of the second connecting arm may be a center of the second connecting arm.

Furthermore, a length of section L from a rotation center to one end of each of the first connecting arm and the second connecting arm may be the same as a length of section R from the rotation center to an opposite end of each of the first connecting arm and the second connecting arm.

Furthermore, a first linear stage and a second linear stage, to which the first slider and the second slider are coupled to be linearly moved, respectively, may be included.

Furthermore, a counter mass that is detachably coupled to the second slider may be further included.

Furthermore, a mass of the counter mass may be set such that a total mass of all transfer elements located in a direction, in which the scanning head is positioned, becomes equal to a total mass of all transfer elements located in an opposite direction.

### [ADVANTAGEOUS EFFECTS OF THE INVENTION]

According to the aforementioned solution of the present disclosure, it is possible to solve all of the problems of nonuniform light transmission caused by bending of a guiding optical fiber, mechanical resistance generated by the guiding optical fiber, a narrow scanning range that may occur in conventional methods, and mechanical vibration regardless of the scanning range (stroke) during high-speed scanning.

In addition, according to the aforementioned solution of the present disclosure, when the mechanical scanner structure of the present disclosure is applied to a photoacoustic imaging device, a laser beam of highly uniform intensity may be delivered to a target tissue regardless of the scanning range and speed, thereby improving the reliability of the provided photoacoustic image, and, because the elastic resistance generated by the optical fiber in conventional scanning structures may be completely eliminated, much faster image scanning may be achieved over a significantly wider area than before.

In addition, the increased light intensity uniformity achieved by the photoacoustic scanner structure of the present disclosure greatly enhances the reliability of quantitative imaging, which has recently become important in this field, by improving the quantitative accuracy of the photoacoustic image provided during functional photoacoustic imaging processes requiring spectroscopic imaging, such as dual-wavelength imaging.

Effects of the present disclosure are not limited to the effects mentioned above, and other effects not mentioned will be clearly understood by those skilled in the art from the following description.

### [DESCRIPTION OF THE DRAWINGS]

FIG. 1 is a schematic diagram of a photoacoustic scanner according to an embodiment of the present disclosure.
FIG. 2 is a view illustrating an operation of the photoacoustic scanner of FIG. 1.
FIG. 3 is a schematic diagram of a photoacoustic scanner according to another embodiment of the present disclosure.
FIG. 4 is a schematic diagram of a photoacoustic scanner according to still another embodiment of the present disclosure.
FIG. 5 is a schematic diagram of a scanning head according to an embodiment of the present disclosure.
FIG. 6 is a schematic diagram of a scanning head according to another embodiment of the present disclosure.
FIG. 7 is a schematic diagram of a scanning head according to still another embodiment of the present disclosure.
FIG. 8 is a schematic diagram of a photoacoustic scanner according to still another embodiment of the present disclosure.
FIG. 9 is a view illustrating the photoacoustic scanner of FIG. 8, viewed from a top.
FIG. 10 is a view illustrating movement directions of a scanning head and a counter mass of the photoacoustic scanner of FIG. 8.
FIG. 11 is a schematic diagram illustrating an example, in which a counter mass may be mounted to minimize a total weight of a photoacoustic scanner according to still another embodiment of the present disclosure.
FIG. 12 is a schematic diagram illustrating a situation, in which an encoder capable of measuring a precise translation value of a scanning head in a high-speed scanning process of a photoacoustic scanner according to still another embodiment of the present disclosure is mounted.
FIG. 13 is a schematic diagram illustrating a structure of a wide-range vibration-free linear high-speed reciprocating scanning device capable of completely eliminating mechanical vibration problems during wide-range high-speed reciprocating scanning, according to an embodiment of the present disclosure.
FIG. 14 is a perspective view for more clearly illustrating a shape of a crank applied to FIG. 13.
FIG. 15 is a schematic diagram illustrating structures and shapes of a first link and a second link applied to FIGS. 8 to 13.
FIG. 16 is a schematic diagram illustrating an operation principle of the embodiment presented in FIG. 13.

### [BEST MODE]

The same reference numerals denote the same elements throughout the present disclosure. The present disclosure does not describe all elements of embodiments. Well-known content or redundant content in which embodiments are the same as one another will be omitted in a technical field to which the present disclosure belongs. A term such as 'unit, module, member, or block' used in the specification may be implemented with software or hardware. According to embodiments, a plurality of 'units, modules, members, or blocks' may be implemented with one component, or a single 'unit, module, member, or block' may include a plurality of components.

Throughout this specification, when it is supposed that a portion is "connected" to another portion, this includes not only a direct connection, but also an indirect connection. The indirect connection includes being connected through a wireless communication network.

Furthermore, when a portion "comprises" a component, it will be understood that it may further include another component, without excluding other components unless specifically stated otherwise.

Throughout this specification, when it is supposed that a member is located on another member "on", this includes not only the case where one member is in contact with another member but also the case where another member is present between two other members.

Terms such as first, second, and the like are used to distinguish one component from another component, and thus the component is not limited by the terms described above.

Unless there are obvious exceptions in the context, a singular form includes a plural form.

In each step, an identification code is used for convenience of description. The identification code does not describe the order of each step. Unless the context clearly states a specific order, each step may be performed differently from the specified order.

Hereinafter, an operation principle and embodiments of the present disclosure will be described with reference to the accompanying drawings.

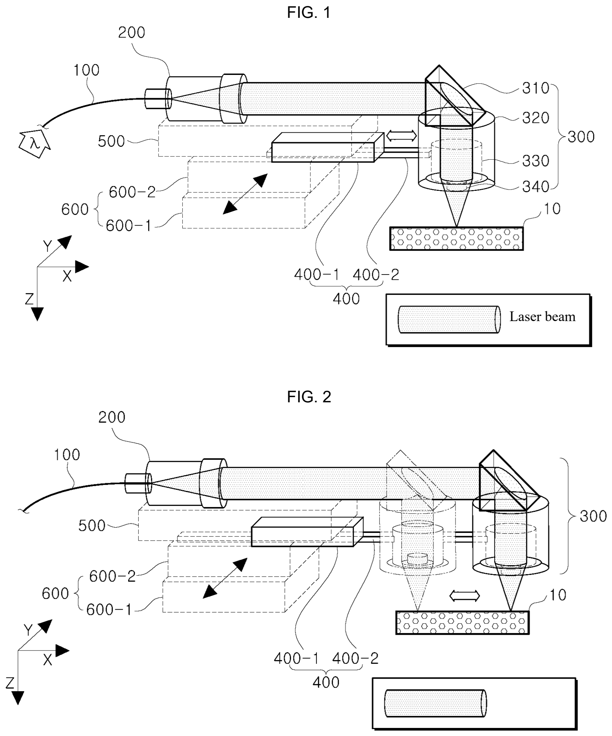

FIG. 1 is a schematic diagram of a photoacoustic scanner according to an embodiment of the present disclosure.

A photoacoustic scanner according to an embodiment may include a collimator 200, a scanning head 300, a linear actuator 400, a base frame 500, and a stage 600, as illustrated in FIG. 1. Here, the scanning head 300 may include a prism 310, an illumination optical part 330, an ultrasonic transducer 340, and a scanning head frame 320.

Hereinafter, the above-described components will be described in detail as follows.

The collimator 200 has a structure that may be removably attached to a guiding optical fiber 100, and may receive light from a light source (not illustrated) through the guiding optical fiber 100, and the received light may exit as a collimated light. For example, the collimator 200 may receive a laser beam from a light source through the guiding optical fiber 100 and generate collimated light, and the generated collimated light may be provided to a prism 310 of the scanning head 300. In other words, the collimator 200 may serve to emit (or modulate) the light provided from a light source through the guiding optical fiber 100 to the prism 310, in a form of a precise collimated light. In an embodiment, the light source may be a pulsed light source used for a photoacoustic image.

The prism 310 may switch a direction of the collimated light that exits from the collimator 200. For example, the prism 310 may change a travel path of the collimated light that exits from the collimator 200 so that the collimated light from the collimator 200 may be incident on the scanning head 300. According to an embodiment, light (for example, collimated light) that exits from the collimator 200 along the +X-axis direction may be incident on the prism 310 in the +X-axis direction, and a direction of the light that is incident on the prism 310 in the +X-axis direction may be switched by 90° with respect to the +X-axis direction, and the light may exit in the +Z-axis direction.

The illumination optical part 330 may serve to induce a photoacoustic signal by illuminating light to a target tissue 10 according to a specific illumination pattern. To this end, according to an embodiment, the illumination optical part 330 may include at least one (for example, a single or a plurality of) lens.

The ultrasonic transducer 340 may detect a photoacoustic wave that is induced by the illumination optical part 330.

The scanning head frame 320 may fix the prism 310, the illumination optical part 330, and the ultrasonic transducer 340 described above. For example, the scanning head frame 320 may serve as a frame that integrally fixes the prism 310, the illumination optical part 330, and the ultrasonic transducer 340 described above.

The linear actuator 400 may be connected to the scanning head 300 to provide a precise linear motion condition to the scanning head 300, and may control reciprocation thereof. For example, the linear actuator 400 may generate a linear reciprocating motion along the +X-axis direction and an opposite direction (hereafter, the -X-axis direction) to the +X-axis direction. The linear actuator 400 may provide power that is necessary for a linear reciprocating motion, to the scanning head 300. To this end, according to an embodiment, the linear actuator 400 may be connected to the scanning head 300. For example, the linear actuator 400 may be attached to an outer peripheral surface of the scanning head 300. According to an embodiment, the linear actuator 400 may include a fixed part 400-1 and a movable part 400-2 (for example, an arm). A driving motor (not illustrated) may be disposed in an interior of the fixed part 400-1, and power of the driving motor may be transmitted to the movable part 400-2. The movable part 400-2 may perform a linear reciprocating motion in the +X-axis direction or the -X-axis direction in response to a driving force provided from the driving motor. The movable part 400-2 of the linear actuator 400 may be attached to the scanning head 300 described above. When the movable part 400-2 of the linear actuator 400 is moved (for example, is extended) along the +X-axis direction, the scanning head 300 attached to the movable part 400-2 may be moved along the +X-axis direction. For example, when the movable part 400-2 of the linear actuator 400 is extended in the +X-axis direction, all the components (for example, the prism 310, the scanning head frame 320, the illumination optical part 330, and the ultrasonic transducer 340) of the scanning head 300 may be moved together along the +X-axis direction. Meanwhile, when the movable part 400-2 of the linear actuator 400 is moved (for example, is contracted) along the -X-axis direction, the scanning head 300 attached to the movable part 400-2 may be moved along the -X-axis direction. For example, when the movable part 400-2 of the linear actuator 400 is contracted in the -X-axis direction, all the components (for example, the prism 310, the scanning head frame 320, the illumination optical part 330, and the ultrasonic transducer 340) of the scanning head 300 may be moved together along the -X-axis direction.

The base frame 500 may fix the collimator 200 and the linear actuator 400 to each other. For example, the collimator 200 may be disposed on an upper surface of the base frame 500, and in this case, the collimator 200 may be attached and fixed to the upper surface of the base frame 500. Furthermore, the linear actuator 400 may be inserted into an interior of the base frame 500 to be fixed. For example, the fixed part 400-1 of the linear actuator 400 may be inserted into the interior of the base frame 500 to be fixed to the base frame 500. Accordingly, the collimator 200 and the linear actuator 400 may be fixed to the base frame 500. In this case, the fixed part 400-1 of the linear actuator 400 may be attached and fixed to the base frame 500, whereas the movable part 400-2 of the linear actuator 400 may be attached to and fixed to the scanning head 300 described above. For example, one end of the movable part 400-2 may be attached and fixed to an outer peripheral surface of the scanning head 300.

The stage 600 may be disposed at a lower portion of the base frame 500. Accordingly, the base frame 500 may be disposed between the collimator 200 and the stage 600. Furthermore, the linear actuator 400 may also be disposed between the collimator 200 and the stage 600. The stage 600 may be attached to and fixed to the base frame 500 described above. The stage 600 may perform a linear reciprocating motion along a direction that crosses a movement direction (for example, the +X-axis direction and/or the -X-axis direction) of the linear actuator 400 described above. For example, the stage 600 may be moved along the +Y-axis direction and an opposite direction (hereinafter, the -Y-axis direction) to the +Y-axis direction. According to an embodiment, the stage 600 may be a motorized Y-stage. To this end, the stage 600 according to an embodiment may include a support plate 600-1 and a movable plate 600-2. The movable plate 600-2 may be disposed on the support plate 600-1. The movable plate 600-2 may be driven by a driving motor inside the support plate 600-1, and may be moved along the +Y-axis direction and the -Y-axis direction described above. The movable plate 600-2 of the above-described stage 600 may be attached to a lower surface of the above-described base frame 500. As the stage 600 is moved along the +Y-axis direction and the -Y-axis direction, the base frame 500 connected to the stage 600 may be moved along the +Y-axis direction and the -Y-axis direction, and as the base frame 500 is moved along the +Y-axis direction and the -Y-axis direction, the linear actuator 400 and the collimator 200 connected to the base frame 500 may be moved along the +Y-axis direction and the -Y-axis direction, and as the linear actuator 400 is moved along the +Y-axis direction and the -Y-axis direction, the scanning head 300 connected to the movable part 400-2 of the linear actuator 400 may be moved along the +Y-axis direction and the -Y-axis direction.

In this way, as the linear actuator 400 is moved in the +X-axis direction and the -X-axis direction, the scanning head 300 may be moved (or transferred) along the +X-axis direction and the -X-axis direction, and as the stage 600 is moved in the +Y-axis direction and the -Y-axis direction, the scanning head 300 may be moved (or transferred) along the +Y-axis direction and the -Y-axis direction. In other words, the scanning head 300 may be moved (or transferred) in the +X-axis direction and the -X-axis direction, and the +Y-axis direction and the -Y-axis direction by the linear actuator 400 and the stage 600.

Consequently, according to the photoacoustic scanner of an embodiment, as the scanning head 300 is moved in the +X-axis direction and the -X-axis direction by a reciprocating linear motion provided by the linear actuator 400, a two-dimensional photoacoustic image (for example, a B-scan image) on the X-Z plane may be acquired, and through a scan motion in the +Y-axis direction and the -Y-axis direction additionally provided by the stage 600, a final three-dimensional volume image for a target tissue 10 may be acquired. Of course, in still another embodiment, the stage 600 described above does not necessarily have to be a stage that provides only a linear motion along the +Y-axis direction and the -Y-axis direction, and may be replaced with a device that provides a curved motion, such as an arc, like a goniometer, or a rotational motion, like a rotational stage. Of course, when this element is applied, the resulting three-dimensional image to be provided may have a shape like a cylinder.

FIG. 2 is a view illustrating an operation of the photoacoustic scanner of FIG. 1. For example, FIG. 2 is a schematic diagram illustrating how a position of the scanning head 300 of the photoacoustic scanner of FIG. 1 is changed in a process of performing reciprocal scanning along the +X-axis direction and the -X-axis direction by the scanning head 300, according to an operation principle of the photoacoustic scanner of the above-described embodiment.

Referring to FIG. 2, the linear actuator 400 may include a fixed part 400-1 that is fixed to the base frame 500, and a movable part 400-2 that is attached to the scanning head 300 to perform a reciprocating motion (for example, a reciprocating motion in the +X-axis direction and the -X-axis direction), and a transfer range (for example, a stroke) of the linear actuator 400 may determine a range that may be actually scanned by physical movement. For example, a range of motion of the movable part 400-2 provided in the linear actuator 400, in the +X-axis direction and the -X-axis direction, may define a scannable range of the photoacoustic scanner according to an embodiment, in the +X-axis direction and the -X-axis direction.

Meanwhile, it may be understood that a fixed part 400-1 and a movable part 400-2 of the linear actuator 400 provided in the photoacoustic scanner according to an embodiment correspond to, for example, a block part and a rail part provided in a linear motion (LM) guide that is utilized in the industrial field, but the linear actuator 400 according to an embodiment is not limited thereto. For example, the fixed part 400-1 provided in the photoacoustic scanner according to an embodiment may be a device or a means of a concept including all power providing devices, such as a motor.

As illustrated in FIG. 2, in a state in which the stage 600 is stopped (for example, in a state in which a motion of the scanning head 300 in the +Y-axis direction and the -Y-axis direction is stopped), the linear actuator 400 may perform a reciprocating linear motion (for example, a motion in the +X-axis direction and the -X-axis direction) alone to acquire a B-scan image for the target tissue 10.

According to an embodiment, the linear actuator 400 includes the fixed part 400-1 and the movable part 400-2, and during a reciprocating motion (for example, a linear reciprocating motion in the +X-axis direction and the -X-axis direction) of the movable part 400-2, only the scanning head 300 is moved and the collimator 200 is not moved, so that the guiding optical fiber 100 fastened to the collimator 200 is not moved either. In other words, because the collimator 200 is maintained in a fixed state even during a scanning operation in the +X-axis direction and the -X-axis direction for acquiring a B-scan image, the guiding optical fiber 100 connected to the collimator 200 may be maintained in a specific shape without being bent. Accordingly, a bending problem of the guiding optical fiber 100 may be resolved during a high-speed scanning process, and consequently, in a process of obtaining a two-dimensional photoacoustic image along the X-Z plane, an amount of light delivered to the target tissue 10 becomes much more uniform regardless of a scan range in the +X-axis direction and the -X-axis direction, and as a result, a photoacoustic image that is much more quantitatively reliable than that of the conventional technology may be acquired. In addition, a problem of the conventional technology, such as a kind of resistance (for example, a resistance due to bending of the guiding optical fiber 100) that is caused by the guiding optical fiber 100 attached to the collimator 200 may also be removed, so that an additional effect of remarkably fast high-speed scanning may be obtained as compared with the conventional technology. Actually, in the conventional technology, when the guiding optical fiber 100 to be applied to the corresponding device has a thickness of 200 µm or more and is in a multimode form rather than a single-mode form, the resistance problem due to bending of the guiding optical fiber 100 has been a significant issue that could never be ignored.

Meanwhile, to effectively carry out a scanning operation according to a principle of the above-described embodiment, the collimator 200 has to make highly accurate collimated light, and precise optical alignment of laser beams that are incident on the scanning head 300 from the collimator 200 has to be secured. However, in the field of optical fiber technology, precision collimators 200 for optical fibers based on aspherical lenses are already commercially available, and the current machining and assembly technologies for related mechanical frames are at a level that poses no difficulty in achieving precise axial alignment between the collimator 200 and the scanning head 300. Therefore, a photoacoustic scanner capable of performing a scanning operation according to a principle of an embodiment is a sufficiently realizable (or practicable) device at the present stage.

Of course, even if the photoacoustic scanner is implemented according to an embodiment, it is true that a signal transmission wire (not illustrated), which is typically required to be attached to the ultrasonic transducer 340, is not completely removed, but, because the signal transmission wire is not a factor that affects the non-uniformity problem of the photoacoustic image raised in the embodiment, and because a thickness of the signal transmission wire required for the related device may be 200 µm or less without any issue, a bending problem of the wire during a reciprocating motion does not occur at all.

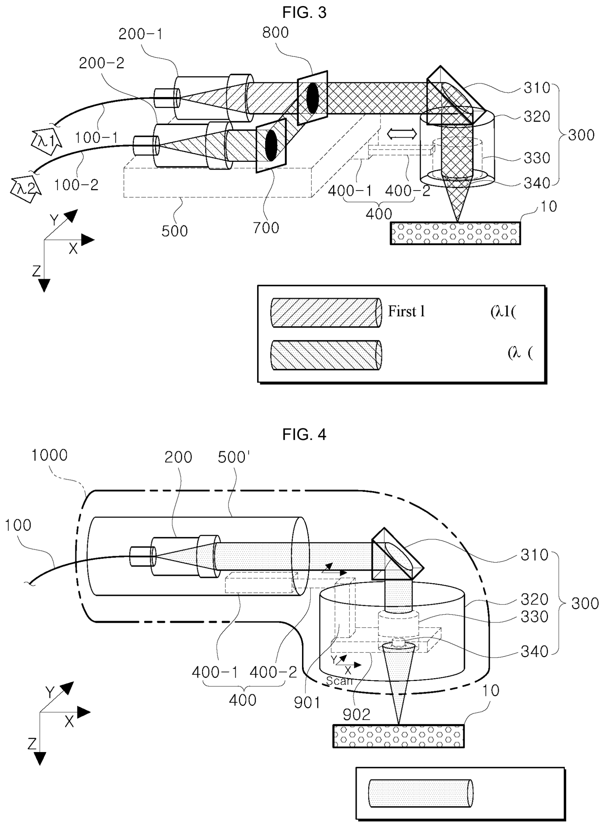

An embodiment of the photoacoustic scanner, to which a single-wavelength laser beam is applied, has been presented above. However, the concept of the photoacoustic scanner presented in the disclosure according to an embodiment may be sufficiently expanded and implemented in a form using two or more wavelengths. A photoacoustic scanner according to an embodiment for this will be described in detail below with reference to FIG. 3.

FIG. 3 is a schematic diagram of a photoacoustic scanner according to another embodiment of the present disclosure.

As illustrated in FIG. 3, a photoacoustic scanner according to an embodiment may include a first collimator 200-1, a second collimator 200-2, a reflection mirror 700, a beam combiner 800, a scanning head 300, a linear actuator 400, and a base frame 500. Here, the scanning head 300 may include a prism 310, an illumination optical part 330, an ultrasonic transducer 340, and a scanning head frame 320.

The first collimator 200-1 may receive a first laser beam (for example, a laser beam of a first wavelength λ1) from a first light source (not illustrated) through a first guiding optical fiber 100-1, and may make the received light into collimated light and exit it. For example, the first collimator 200-1 may receive a first laser beam from a first light source through the first guiding optical fiber 100-1 to generate collimated light (hereinafter referred to as a first collimated light), and may deliver the generated first collimated light to the beam combiner 800. In an embodiment, the first light source may be a pulsed light source used for a photoacoustic image.

The second collimator 200-2 may be disposed adjacent to the first collimator 200-1. For example, the second collimator 200-2 may be disposed adjacent to the first collimator 200-1 in the -Y-axis direction. In addition, the second collimator 200-2 and the first collimator 200-1 may be disposed in parallel to each other along the X-axis direction. The second collimator 200-2 may receive a second laser beam (for example, a laser beam of a second wavelength λ2) from a second light source (not illustrated) through a second guiding optical fiber 100-2, and may make the received light into collimated light and exit it. For example, the second collimator 200-2 may receive a second laser beam from a second light source through the second guiding optical fiber 100-2 to generate collimated light (hereinafter referred to as a second collimated light), and may deliver the generated second collimated light to the reflection mirror 700. In an embodiment, the second light source may be a pulsed light source used for a photoacoustic image. The above-described first wavelength λ1 is a fundamental wavelength, and the second wavelength λ2 is an additional wavelength, and the first wavelength λ1 and the second wavelength λ2 may have different values from each other.

The reflection mirror 700 may reflect the second collimated light incident from the second collimator 200-2 and provide it to the above-described beam combiner 800. In other words, a travel path of the second collimated light from the second collimator 200-2 may be changed by the reflection mirror 700, and may be incident on the beam combiner 800.

The beam combiner 800 may combine the first collimated light from the first collimator 200-1 and the second collimated light from the second collimator 200-2. For example, by the beam combiner 800, the first collimated light from the first collimator 200-1 and the second collimated light from the second collimator 200-2 may overlap each other exactly. The collimated lights overlapped by the beam combiner 800 may be incident on a prism 310 of the scanning head 300 in an accurate collimated light form.

Since the configuration and operation of the scanning head 300 of FIG. 3 are the same as those of the scanning head 300 of FIG. 1 described above, a description of the scanning head 300 of FIG. 3 will refer to the scanning head 300 of FIG. 1 and the related contents described above.

In the embodiment of FIG. 3 as well, the key point is to make the collimated lights emitted from the two collimators, that is, the first collimator 200-1 and the second collimator 200-2, exactly overlap each other by using the beam combiner 800, and to perfectly align them so that they are accurately incident on the prism 310 mounted on the scanning head 300, and, to make the laser beam of the additional wavelength λ2 emitted from the second collimator 200-2 into a perfect collimated light, a precise aspherical lens that is suitable for the corresponding wavelength has to be applied. Of course, in FIG. 3, the second guiding optical fiber 100-2 may serve to deliver the second laser beam of the additional laser wavelength λ2 from a corresponding light source (for example, a second light source) to the second collimator 200-2.

Meanwhile, the photoacoustic scanner of FIG. 3 may not include the stage 600 illustrated in FIG. 1. However, unlike this, the photoacoustic scanner of FIG. 3 may further include the above-described stage 600 (for example, a motorized Y-stage). For example, the above-described stage 600 may be further attached to a lower portion of the base frame 500 of FIG. 3.

As described above with reference to FIG. 3, an embodiment of the photoacoustic scanner, to which two laser beams having different wavelengths are applied, has been explained. However, the described principle of wavelength addition may be extended not only to two wavelengths but also to three or more wavelengths, and may also be sufficiently extended and applied when an acoustic-resolution photoacoustic imaging mode, which mainly applies a weakly focused laser beam, is to be simultaneously integrated and implemented in a single device. Here, the latter (for example, an embodiment in which an acoustic-resolution photoacoustic imaging mode that mainly applies a weakly focused laser beam is simultaneously integrated into a single device) refers to a case, in which both an optical-resolution photoacoustic imaging mode and an acoustic-resolution photoacoustic imaging mode are integrally implemented within one device. Of course, when the acoustic-resolution photoacoustic imaging mode is added, it is theoretically impossible to make light emitted from a multimode optical fiber, which has to generally be applied to that mode, into a perfectly collimated light, but, due to the nature of the acoustic-resolution photoacoustic imaging mode, a slight non-parallelism does not cause a significant problem.

FIG. 4 is a schematic diagram of a photoacoustic scanner according to still another embodiment of the present disclosure.

As illustrated in FIG. 4, a photoacoustic scanner according to an embodiment may include a case 1000, a collimator 200, a scanning head 300, a linear actuator 400, and a housing 500'. Here, the scanning head 300 may include a prism 310, an illumination optical part 330, an ultrasonic transducer 340, and a scanning head frame 320.

The case 1000 may be surrounded by the collimator 200, the scanning head 300, the linear actuator 400, and the housing 500' described above. Of course, the case 1000 may include a portion that has a handle shape.

The collimator 200 and the linear actuator 400 may be disposed in an interior of the housing 500'. In this case, the collimator 200 and the linear actuator 400 may be fixed to the housing 500' in the interior of the housing 500'. For example, the collimator 200 and the linear actuator 400 may be attached and fixed to an inner wall of the interior of the housing 500'. Here, as described above, the linear actuator 400 may include the fixed part 400-1 and the movable part 400-2, and the fixed part 400-1 of the linear actuator 400 may be attached to an inner wall of the housing 500'.

A first support part 901 may be disposed in an interior of the scanning head frame 320. The first support part 901 may be connected to the movable part 400-2 of the linear actuator 400. For example, one end of the first support part 901 may be coupled to one end of the movable part 400-2. The first support part 901 may extend in a direction (for example, the Z-axis direction) that crosses an extension direction (for example, the X-axis direction) of the movable part 400-2. Furthermore, the first support part 901 may be attached and fixed to the inner wall of the scanning head frame 320 in the interior of the scanning head frame 320. For example, the first support part 901 may be fixed to an inner wall of the scanning head frame 320.

A second support part 902 may be disposed in an interior of the scanning head frame 320. The second support part 902 may be connected to the first support part 901. For example, one end of the second support part 902 may be coupled to an opposite end of the first support part 901. Meanwhile, the second support part 902 and the first support part 901 may be formed integrally. The first support part 901 and the second support part 902 formed integrally may have an L-shaped cross-section. The second support part 902 may extend in a direction (for example, the X-axis direction) that crosses an extension direction (for example, the Z-axis direction) of the first support part 901. The second support part 902 may surround the illumination optical part 330. To this end, according to an embodiment, the second support part 902 may have a hole, through which the illumination optical part 330 passes to be inserted. The illumination optical part 330 may be surrounded by the hole of the second support part 902 while passing through the hole. In this case, the illumination optical part 330 may be attached and fixed to an inner wall of the hole of the second support part 902.

When the movable part 400-2 of the linear actuator 400 is moved (for example, is extended) along the X-axis direction, the first support part 901 and the second support part 902 attached to the movable part 400-2 may be moved along the X-axis direction. Furthermore, as the first support part 901 and the second support part 902 are moved along the X-axis direction, the scanning head 300 attached to the first support part 901 and the second support part 902 may be moved in the X-axis direction.

For example, when the movable part 400-2 of the linear actuator 400 is extended in the +X-axis direction, all the components (for example, the prism 310, the scanning head frame 320, the illumination optical part 330, and the ultrasonic transducer 340) of the scanning head 300 may be moved together along the -X-axis direction. Meanwhile, when the movable part 400-2 of the linear actuator 400 is moved (for example, is contracted) along the -X-axis direction, the first support part 901 and the second support part 902 attached to the movable part 400-2 may be moved along the -X-axis direction. Furthermore, as the first support part 901 and the second support part 902 are moved along the -X-axis direction, the scanning head 300 attached to the first support part 901 and the second support part 902 may be moved in the -X-axis direction. For example, when the movable part 400-2 of the linear actuator 400 is contracted in the -X-axis direction, all the components (for example, the prism 310, the scanning head frame 320, the illumination optical part 330, and the ultrasonic transducer 340) of the scanning head 300 may be moved together along the -X-axis direction.

Meanwhile, the photoacoustic scanner of FIG. 4 may further include the stage 600 (for example, a motorized Y-stage) illustrated in FIG. 2, which is attached to the housing 500'. When the stage 600 is moved along the +Y-axis direction and the -Y-axis direction, the housing 500' attached to the stage 600 may be moved along the +Y-axis direction and the - Y-axis direction. Furthermore, when the housing 500' is moved along the +Y-axis direction and the -Y-axis direction, the collimator 200 and the linear actuator 400 in the interior of the housing 500' may be moved together along the +Y-axis direction and the -Y-axis direction. Furthermore, when the linear actuator 400 is moved along the +Y-axis direction and the -Y-axis direction, the first support part 901 and the second support part 902 connected to the movable part 400-2 of the linear actuator 400 may be moved together along the +Y-axis direction and the -Y-axis direction. Furthermore, when the first support part 901 and the second support part 902 are moved along the +Y-axis direction and the -Y-axis direction, the scanning head 300 connected to the first support part 901 and the second support part 902 may be moved together along the +Y-axis direction and the -Y-axis direction. Furthermore, when the scanning head 300 is moved along the +Y-axis direction and the -Y-axis direction, the prism 310, the illumination optical part 330, the ultrasonic transducer 340, and the scanning head frame 320 of the scanning head 300 may be moved together along the +Y-axis direction and the -Y-axis direction.

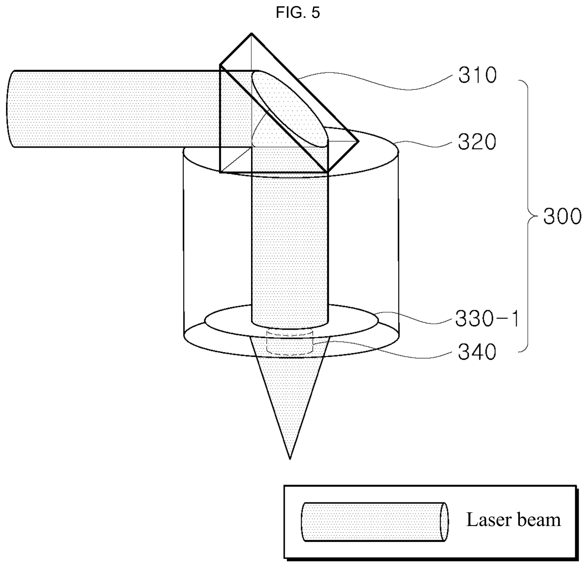

Meanwhile, the structure of the scanning head 300 illustrated in FIG. 1 may be implemented in various other forms, as exemplified in other embodiments illustrated in FIGS. 5 to 7. Hereinafter, various modifiable embodiments of the scanning head 300 will be described in detail with reference to FIGS. 5 to 7.

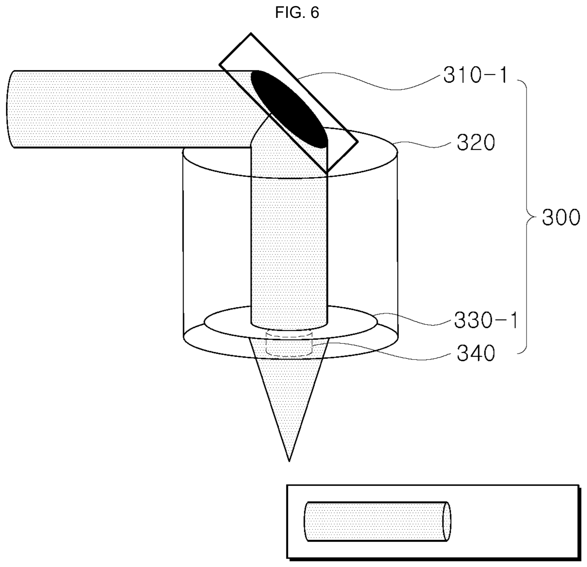

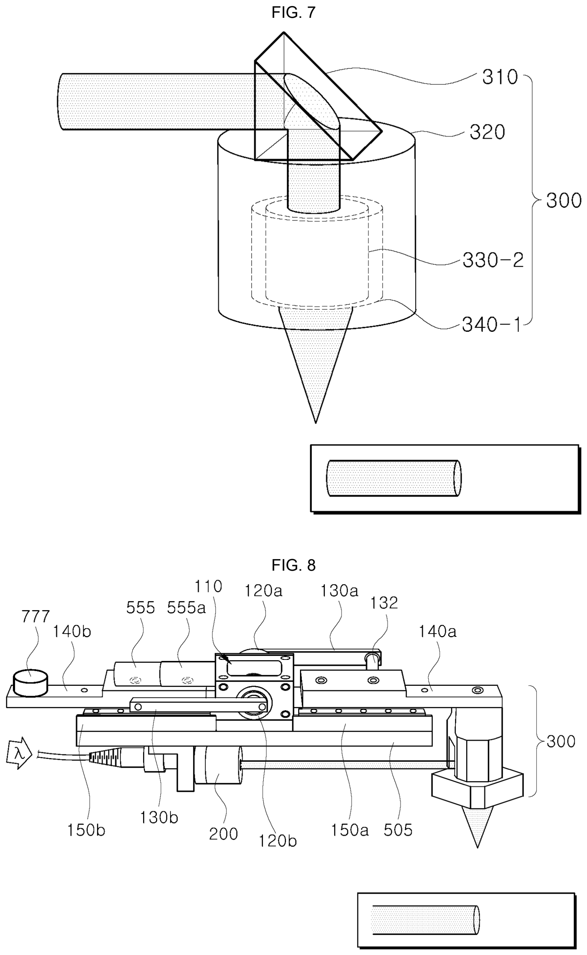

FIG. 5 is a schematic diagram of the scanning head 300 according to an embodiment of the present disclosure, FIG. 6 is a schematic diagram of the scanning head 300 according to another embodiment of the present disclosure, and FIG. 7 is a schematic diagram of the scanning head 300 according to another embodiment of the present disclosure.

For example, the illumination optical part 330 may be implemented in a very simple form by applying a single convex lens 330-1 that performs a light-converging function, as illustrated in FIG. 5, and in this case, it is preferable that the ultrasonic transducer 340 is located at a central lower point of the single convex lens 330-1. Here, the ultrasonic transducer 340 is a component that serves to detect a photoacoustic wave induced by a laser pulse delivered to the target tissue 10, and in the case of an optical-resolution photoacoustic image mode, the lateral resolution is generally determined by a beam diameter of the laser beam at the focus and thus, its diameter may be less than 1 mm, which may be small enough not to significantly interfere with the laser beam that propagates around it. Furthermore, because a desired image may be generated by applying a reconstruction principle even if an acoustic-resolution photoacoustic image mode that does not focus the laser beam delivered to the target tissue 10 is applied, a diameter of the ultrasonic transducer 340 to be applied does not necessarily have to be greater than 1 mm. A principle for setting dimensions related to a diameter of the ultrasonic transducer 340 and a diameter of a laser beam that passes around it will be omitted, as it is already obvious in the relevant field.

In the case of the prism 310 that changes a direction of collimated light emitted from the collimator 200 by 90 degrees and provides it to the illumination optical part 330, a simple flat mirror 310-1 may be applied, as illustrated in FIG. 6, and positions of the illumination optical part 330 and the ultrasonic transducer 340 may also be switched, as illustrated in FIG. 7. Here, the GRIN lens 330-2 illustrated in FIG. 7 has a cylindrical shape, but it has a characteristic, in which a refractive index inside the lens decreases as it becomes more distant from a central axis, and thus, when collimated light enters through its incident surface, the lens functions to converge the light while it passes through the interior. In this way, when the GRIN lens 330-2 is used as the illumination optical part 330, the ultrasonic transducer 340 that detects a photoacoustic wave may be replaced with a ring transducer 340-1 having a ring-shaped aperture, as illustrated in FIG. 7.

According to an embodiment, the scanning head 300 of FIG. 1 may be replaced with any one of the scanning heads 300 of FIGS. 5 to 7. Likewise, the scanning head 300 of FIG. 3 may be replaced with any one of the scanning heads 300 of FIGS. 5 to 7. Likewise, the scanning head 300 of FIG. 4 may be replaced with any one of the scanning heads 300 of FIGS. 5 to 7.

According to the photoacoustic scanner of an embodiment, all of the following problems that have long been raised as troublesome issues in the related art, which are a problem of non-uniform light delivery caused by bending of the guiding optical fiber 100 directly connected to the scanning head 300 during mechanical scanning, a problem of mechanical resistance generated by the guiding optical fiber 100, and a problem of a narrow scanning range in the related art, may be solved.

According to an embodiment, the improved uniformity of light intensity may greatly contribute to enhancing the quantitative reliability of photoacoustic images provided during a functional photoacoustic imaging process that requires spectroscopic imaging, such as dual-wavelength imaging, and the scanning concept proposed in the embodiment may be implemented in the form of a handle-type, that is, a handheld probe, which allows a user to freely image a desired region of a subject's body surface, such as a human or animal, by directly holding the device by hand, and moreover, it is apparent that it may also be implemented in a wide variety of forms, such as a miniature probe for imaging the interior of a living body near the body surface in either an invasive or non-invasive manner, accompanied by surgery, and even as an endoscopic device for imaging deep areas within a living body.

Hereinafter, with reference to FIGS. 8 to **11****,** the present photoacoustic scanner, which has an innovative structure capable of eliminating mechanical vibrations that may occur during a high-speed scanning process by offsetting the total momentum vector of all moving elements of the device so as to become zero even during a reciprocating motion of the scanning head 300, will be described in detail.

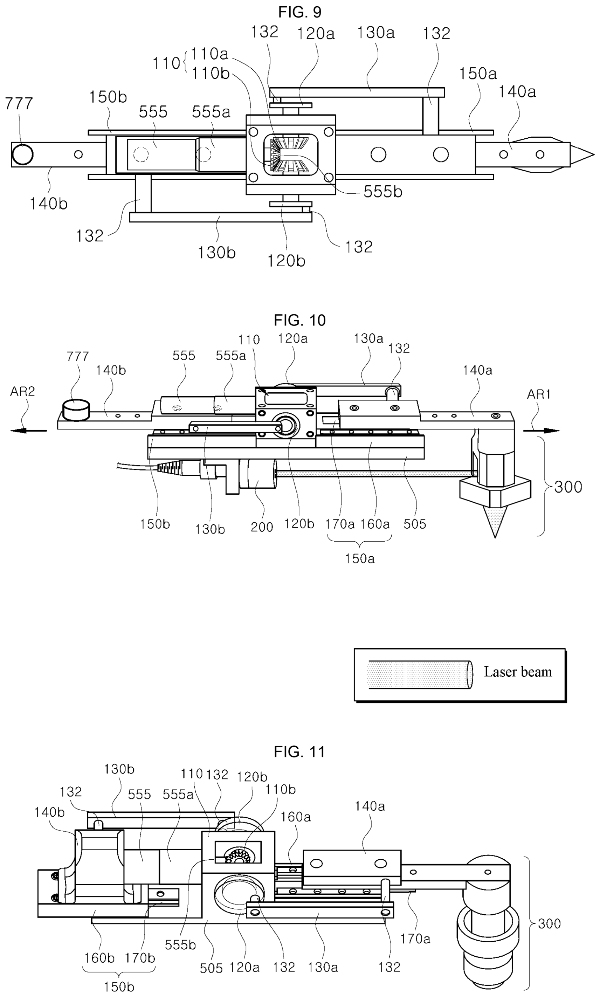

FIG. 8 is a schematic diagram of a photoacoustic scanner according to still another embodiment of the present disclosure, and FIG. 9 is a view of the photoacoustic scanner of FIG. 8, viewed from a top.

The photoacoustic scanner according to an embodiment may include, as illustrated in FIGS. 8 and 9, a collimator 200, a driving motor 555, a frame 505, a scanning head 300, a symmetric actuator, and a counter mass 777 that offsets a total momentum of a transfer part during a scanning process together with the scanning head 300 so that the total momentum becomes zero.

The collimator 200 may receive a laser beam from a light source through the guiding optical fiber and generate collimated light, and the generated collimated light may be provided to the prism of the scanning head 300.

The driving motor 555 is a device that generates rotational power, and a driving motor speed reducer 555a may be additionally provided to increase its torque.

A symmetric actuator may be disposed on the frame 505.

The symmetric actuator may move the scanning head 300 and the counter mass 777 in opposite directions. The symmetric actuator described above may include, as illustrated in FIGS. 8 to 10, a driving device 110, a first rotation part 120a, a first link 130a, a first slider 140a, a first linear stage 150a, a second rotation part 120b, a second link 130b, a second slider 140b, and a second linear stage 150b.

The driving device 110 is a device that receives rotational force from the driving motor speed reducer 555a and distributes and transmits the rotational force to subsequent components, and may include a first bevel gear 110a and a second bevel gear 110b in an interior thereof. Of course, the driving device 110 may alternatively be implemented as a device including the first bevel gear 110a connected to the first rotation part 120a and the second bevel gear 110b connected to the second rotation part 120b, differently from the configuration described above. A central gear 555b may be rotatably connected to a rotary shaft of the driving motor speed reducer 555a, and the first bevel gear 110a and the second bevel gear 110b of the driving device 110 may be coupled to the central gear 555b. Accordingly, when the rotary shaft of the driving motor speed reducer 555a is rotated, the central gear 555b is rotated, and the first bevel gear 110a and the second bevel gear 110b, which are engaged with the central gear 555b, are rotated in opposite directions with the same angular velocity.

A first rotation part 120a may be connected to the first bevel gear 110a of the driving device 110.

The first rotation part 120a may be connected to the first link 130a.

The first link 130a may be connected to the first slider 140a. For example, one side of the first link 130a may be rotatably connected to the first rotation part 120a, and an opposite side of the first link 130a may be connected to the first slider 140a.

Accordingly, when the first bevel gear 110a and the first rotation part 120a are rotated, the connected first link 130a may perform a rotational and translational motion, and the first slider 140a may be moved in a linear direction by a translational motion component of the first link 130a. In this case, the first slider 140a may perform a reciprocating motion in a linear direction together with a first linear stage guide rail 170a, which is a component of the first linear stage 150a (see FIG. 10). Accordingly, when the first bevel gear 110a of the driving device 110 is rotated, the scanning head 300 connected to the first slider 140a may perform linear motion.

The second rotation part 120b may be connected to the second bevel gear 110b of the driving device 110.

The second rotation part 120b may be connected to the second link 130b.

The second link 130b may be connected to the second slider 140b. For example, one side of the second link 130b may be rotatably connected to the second rotation part 120b, and an opposite side of the second link 130b may be connected to the second slider 140b.

Accordingly, when the second bevel gear 110b and the second rotation part 120b are rotated, the connected second link 130b may perform a rotational and translational motion, and the second slider 140b may be moved in a linear direction by a translational motion component of the second link 130b. In this case, the second slider 140b may perform a reciprocating motion in a linear direction together with a second linear stage guide rail 170b, which is a component of the second linear stage 150b (see FIG. 10). Accordingly, when the second bevel gear 110b of the driving device 110 is rotated, the counter mass 777 connected to the second slider 140b may perform a linear motion.

FIG. 10 is a view illustrating movement directions of a scanning head 300 and a counter mass 777 of the photoacoustic scanner of FIG. 8.

Because the first bevel gear 110a and the second bevel gear 110b are rotated in different directions, the first slider 140a and the second slider 140b may be moved in opposite directions. For example, when the first slider 140a is moved in the direction of an arrowhead of a first arrow AR1, the second slider 140b may be moved in the direction of an arrowhead of a second arrow AR2. Accordingly, when the scanning head 300 is moved in the direction of an arrowhead of the first arrow AR1, a center of mass of all moving elements does not move at all. In other words, as the scanning head 300 is moved in the direction of an arrowhead of the first arrow AR1, the counter mass 777 retreats in the direction of an arrowhead of the second arrow AR2, which is opposite to the direction of the first arrow AR1, and thus, a vector sum of momenta of all moving elements, including the scanning head 300 and the counter mass 777, becomes zero. This zero sum of momentum may eliminate the generation of vibrations during high-speed scanning, and thus, the present disclosure may be applicable not only to the described case but also to any device that requires mechanical scanning. Of course, a mass of the counter mass 777 has to be precisely calculated and set so that a total sum of momenta of all moving elements becomes zero.

For reference, in the case of an outer shape of the scanning head 300 illustrated in FIGS. 8 to 10, the example is based on an embodiment in which a lower portion of the scanning head 300 is formed in a streamlined shape like a boat, so as to minimize fluid resistance that may occur during reciprocating motion while the scanning head 300 is submerged in a fluid such as water.

FIG. 11 is a schematic diagram illustrating an example, in which a counter mass may be mounted to minimize a weight of a photoacoustic scanner according to still another embodiment of the present disclosure. That is, by applying another embodiment of the present disclosure as illustrated in FIG. 11, it is also possible to minimize an overall increase in the mass of the device by further reducing a mass value of the counter mass 777, which has to be added to eliminate vibrations generated by the scanning device.

This may be achieved by implementing the installation of the second linear stage 150b, to which the counter mass 777 is mounted, not as in the embodiment illustrated in FIGS. 8 to 10, but as shown in FIG. 11, in which a second linear stage table part 160b is connected to the side of the second slider (in FIG. 11, the counter mass itself serves as the second slider and is therefore not illustrated therein), and the second linear stage guide rail 170b is connected to the frame 505. The reason why simply flipping and installing the corresponding components in this way is meaningful is because, considering that a cross roller table, which is widely used in industry, is a highly effective example of the second linear stage 150b required in the present disclosure, the table portion of the typical cross roller table used in industry is significantly heavier than the rail portion positioned at its center. That is, even with such a simple installation method, it is possible to minimize a mass value of the counter mass 777 to be added, that is, the amount of mass increase. On the other hand, in the case of the first linear stage 150a, to which the scanning head 300 is connected through the first slider 140a, when the aforementioned cross roller table is applied, all dynamic elements including the first slider 140a may be mounted on the first linear stage guide rail 170a rather than on the first linear stage table part 160a, so that their total momentum may be minimized. This is because, in general, the corresponding side (that is, the side, on which the scanning head is located) already has a relatively larger number of components (that is, a larger mass) mounted thereon than the opposite side. Of course, in the embodiment illustrated in FIG. 11, it is assumed that a cross roller table having the aforementioned characteristics is applied, and it should be understood that the second slider 140b, which has a tunnel shape, simultaneously serves as the counter mass 777.

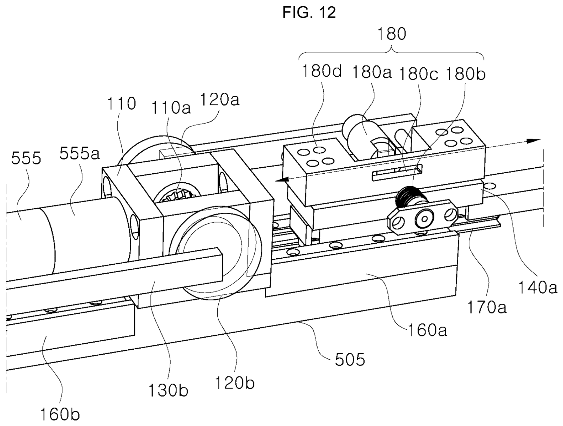

FIG. 12 is a schematic diagram illustrating a state, in which an optical type encoder capable of measuring a precise translation value of a scanning head in a high-speed scanning process of a photoacoustic scanner according to still another embodiment of the present disclosure is mounted. Referring to FIG. 12, an embodiment of a photoacoustic scanner, in which an encoder 180 is mounted to accurately measure a translational displacement of the scanning head 300 and provide related information (signals) when the scanning head 300 performs a reciprocating motion according to the operation principle described above, will be described.

In other words, the encoder 180 is required in a situation where it is necessary to precisely and in real time identify each transfer amount (step) of the scanning head 300 whenever a unit transfer occurs during the movement of the scanning head 300, and, because backlash almost always exists between any two meshed bevel gears in practice, it is generally impossible to accurately detect the rotation angle by mounting a rotary-type encoder inside the driving motor 555.

For this reason, FIG. 12 illustrates an embodiment, in which an encoder 180 that provides an electrical signal (pulse) representing the transferred amount at each transfer step according to an optical method is mounted, and, the encoder 180 is operated on the same principle as a linear encoder of an optical type commonly used in industry, and includes an encoder light source 180a that emits light toward a direction in which the light passes through a linear scale 180c, a photosensor 180b that detects the light having passed through the linear scale 180c, and a linear scale support 180d that fixes the linear scale 180c. Of course, in the embodiments of the present disclosure, only the two components of the linear scale 180c and the linear scale support 180d that fixes the linear scale 180c are mounted on the first slider 140a to perform a linear reciprocating motion together with the first slider 140a, while the other components, namely the encoder light source 180a and the photosensor 180b, are mounted on the frame 505 so as not to be moved, and thus, it is apparent that this configuration is more effective in terms of the total weight of the dynamic parts. The linear scale 180c refers to one component commonly used in an encoder, in which areas that allow light to pass and areas that block light are patterned at regular intervals over a specific range.

Meanwhile, although FIGS. 8 to 12 illustrate a situation in which the first rotation part 120a and the second rotation part 120b have a disk shape, it is apparent that they may also be implemented in a rod shape, like bicycle crank pedals.

A structure of a scanning device, in which the first linear stage and the second linear stage are arranged in a symmetrical form, and motion elements mounted on both sides thereof are configured such that a total center of mass does not move even during a reciprocating linear motion process, thereby eliminating a significant portion of mechanical vibration that may occur during a high-speed scanning operation, has been presented. However, when a device to which this principle is applied is implemented on a large scale of several meters, the size and weight of the first link 130a and the second link 130b also increase accordingly, and in the case of the embodiment illustrated in FIGS. 8 to 12, the vibration contribution generated by them may become significant and cannot be ignored. This is because the first link 130a and the second link 130b, together with the bearing pins 132 connected thereto, are arranged not in a mirror-symmetrical form with respect to the driving device 110, but in an opposite, that is, rotationally symmetrical configuration.

Hereinafter, another embodiment will be provided, which may completely solve the vibration problem that may occur in such a situation, that is, an embodiment concerning a mechanical part that performs a linear reciprocating motion (that is, a scanning device part). In the present specification, the mechanical device is referred to as a "wide-range vibration-free linear reciprocating scanning device" (hereinafter referred to as the vibration-free linear reciprocating scanning device).

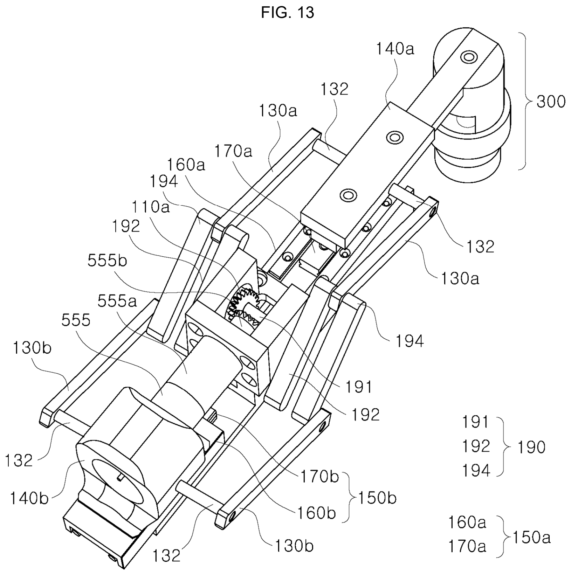

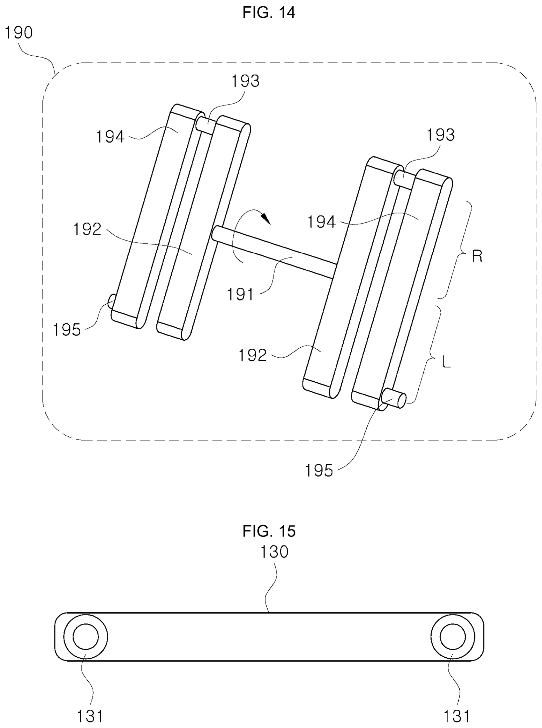

FIG. 13 is a schematic diagram illustrating a structure of a wide-range vibration-free linear reciprocating scanning device that may almost completely eliminate mechanical vibration generated during wide-range high-speed reciprocating scanning according to an embodiment of the present disclosure, FIG. 14 is a perspective view more clearly illustrating a shape of a crank applied to FIG. 13, FIG. 15 is a schematic diagram illustrating structures and shapes of the first link and the second link applied to FIGS. 8 to 13, and FIG. 16 is a schematic diagram for more specifically explaining an operation principle of the embodiment illustrated in FIG. 13.

Referring to FIGS. 13 to 16, a vibration-free linear reciprocating scanning device according to an embodiment of the present disclosure may be implemented in a manner including a first linear stage 150a and a second linear stage 150b disposed on both sides along a longitudinal direction of a frame 505 with reference to a driving device 110, a first slider 140a mounted on a first linear stage guide rail 170a of the first linear stage 150a, a second slider 140b mounted on a second linear stage table part 160b of the second linear stage 150b, two first links 130a connected to left and right sides of the first slider 140a in a mirror-symmetrical direction, two second links 130b connected to left and right sides of the second slider 140b in a mirror-symmetrical direction, and a crank 190 connected to opposite ends of the two first links 130a and the two second links 130b to provide rotational and translational motion forces thereto. That is, in this embodiment, the first rotation part 120a and the second rotation part 120b included in FIGS. 8 to 12 are replaced with a crank 190 having the shape and structure illustrated in FIG. 14, and unlike the embodiment of FIGS. 8 to 12, in which the first bevel gear 110a and the second bevel gear 110b are rotated in opposite directions, in this embodiment, only one bevel gear, that is, the first bevel gear 110a, is involved and installed on the crank 190, more specifically, on a central shaft 191. As a result, opposite side portions of the crank 190, which takes a left-right mirror-symmetrical form with respect to the driving device 110, receive rotational power from a central gear 555b through only the single first bevel gear 110a, so that opposite sides rotate in the same direction.

Of course, for the vibration-free linear reciprocating scanning device provided by the present disclosure to completely eliminate mechanical vibration regardless of the scanning range, the shape and physical characteristics of the crank applied to the device are also very important.

Referring to FIG. 14, the crank 190 has to take a perfectly mirror-symmetrical form with respect to the central shaft 191, on which the first bevel gear 110a is mounted, and the length of section L and the length of section R has to be exactly the same with respect to the central shaft 191, which serves as the center of rotation. That is, the mass distribution on opposite sides of the central shaft 191 has to be exactly the same, and for this purpose, the masses of a first shaft 193, to which the first link 130a is connected, and a second shaft 195, to which the second link 130b is connected, have to also be precisely set so that the mass distribution on the opposite sides is equally formed. The reason this point is important is that, through such an implementation, even if the central shaft 191 performs a high-speed rotational motion, vibration components caused by the centrifugal force of mass points with respect to the central shaft 191 have to be fundamentally prevented from occurring. In addition, because the length of section L and the length of section R are exactly the same with respect to the central shaft 191, when the crank 190 rotates by a certain angle, the first slider 140a and the second slider 140b are moved in opposite directions and by exactly the same distance. Referring again to FIG. 13, in the embodiment of FIG. 13, as in the case of FIG. 11, the second slider 140b having a tunnel shape simultaneously serves as a counter mass 777, and when a mass value of the counter mass 777 is precisely set, the total momentum of all motion elements on the first-direction side and the second-direction side may be set to always be zero (0), regardless of the reciprocating motion range and transfer speed of the first slider 140a and the second slider 140b, so that no mechanical vibration caused by physical motion occurs. Here, two first connecting arms 192 may be connected to each of opposite sides of the central shaft 191. In addition, two first shafts 193 may be connected to the two first connecting arms 192. Two first links 130a may be rotatably coupled to each of the two first shafts 193. In addition, two second connecting arms 194 may be connected to the two first shafts 193. In addition, two second shafts 195 may be connected to each of the two second connecting arms 194. Two second links 130b may be rotatably coupled to each of the two second shafts 195.

FIG. 15 is a schematic diagram illustrating shapes and structures of the first link 130a and the second link 130b applied to FIGS. 8 to 13, and illustrates a structure in which link bearings 131 are provided at opposite ends of the links 130 so that the rotational and translational motion forces provided by the driving device 110 may be properly transmitted to the slider 140 through the links 130.

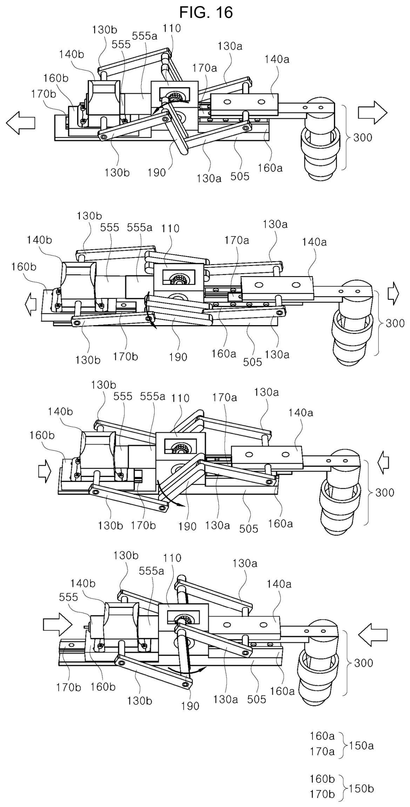

FIG. 16 is a schematic diagram for explaining an operation principle of the embodiment illustrated in FIG. 13, and when the lengths of section L and section R of the crank 190 and the mass distributions of the respective sections are exactly the same, forces required for the linear motions of the first slider 140a and the second slider 140b are evenly transmitted in a left-right balanced manner through the first link 130a and the second link 130b, which are arranged in a mirror-symmetrical form on opposite sides of a longitudinal axis of the frame 505, and thus, when only the mass of the counter mass 777 is accurately calculated and installed, mechanical vibration and centrifugal forces caused by somewhat asymmetrically distributed mass points, as in the embodiments of FIGS. 8 to 10, may theoretically be almost completely eliminated.

A vibration-free linear reciprocating scanning device for transferring a scanning head according to an embodiment of the present disclosure includes a first bevel gear 110a rotated by a driving motor 555, a crank 190 coupled to the first bevel gear 110a to transmit rotational motion of the first bevel gear 110a to a first link 130a and a second link 130b, the first link 130a and the second link 130b connected to the crank 190 to receive power from the crank 190 and perform rotational and linear motions, and a first slider 140a and a second slider 140b respectively connected to the first link 130a and the second link 130b and linearly moved together along linear movement directions of the first link 130a and the second link 130b, and accordingly, a scanning head 300 connected to the first slider 140a may be linearly moved together with the linear motion of the first slider 140a. As an example, the scanning head 300 may be connected to the first slider 140a.

As an example, the crank 190 may include a central shaft 191 coupled to the first bevel gear 110a, two first connecting arms 192 respectively connected to opposite sides of the central shaft 191, two first shafts 193 respectively connected to the two first connecting arms 192 and to which the first link 130a is rotatably coupled, two second connecting arms 194 respectively connected to the two first shafts 193, and two second shafts 195 respectively connected to the two second connecting arms 194 and to which the second link 130b is rotatably coupled.

As an example, a central shaft 191 may be connected to a center of the first connecting arm 192, the second connecting arm 194 may be disposed to be mirror-symmetrical to the first connecting arm 192, a rotation center of the first connecting arm 192 may be located at a center of the first connecting arm 192, and likewise, a rotation center of the second connecting arm 194 may also be located at a center of the second connecting arm 194.

Here, a length of section L from a rotation center of each of the first connecting arm 192 and the second connecting arm 194 to one end thereof may be the same as a length of section R from the rotation center of each of the first connecting arm 192 and the second connecting arm 194 to the opposite end thereof.

A wide-range vibration-free linear reciprocating scanning device according to an embodiment of the present disclosure may further include a first linear stage 150a and a second linear stage 150b, to which the first slider 140a and the second slider 140b are respectively coupled to be linearly movable.

A wide-range vibration-free linear reciprocating scanning device according to an embodiment of the present disclosure may further include a counter mass that is detachably coupled to the second slider 140b. Here, a mass of the counter mass may be determined such that a total mass of components transferred in association with the first slider 140a and a total mass of components transferred in association with the second slider 140b become equal to each other. In other words, a mass of the counter mass may be determined by subtracting a total mass of components moved in association with the second slider 140b from a total mass of components transferred in association with the first slider 140a.

Meanwhile, components transferred in association with the first slider 140a may include the first slider 140a, the first link 130a, the scanning head 300, and section R of the first connecting arm 192 and the second connecting arm 194. In addition, components transferred in association with the second slider 140b may include the second slider 140b, the second link 130b, and section L of the first connecting arm 192 and the second connecting arm 194.

In the present disclosure, a photoacoustic image is set as one specific application example, and a concept of a vibration-free linear reciprocating scanning device capable of completely eliminating mechanical vibration that may occur during linear reciprocating scanning, regardless of a scanning range and speed, has been described together with elements required therefor. However, it will be readily understood by those skilled in the art to which the present disclosure pertains that the presented scanning device may be applied according to the same principle not only to photoacoustic imaging, but also to all other fields requiring linear reciprocating scanning, such as ultrasonic microscopes used to detect defects in large-area LCDs. Disclosed embodiments are described above with reference to the accompanying drawings.

One ordinary skilled in the art to which the present disclosure belongs will understand that the present disclosure may be practiced in forms other than the disclosed embodiments without altering the technical ideas or essential features of the present disclosure. The disclosed embodiments are examples and should not be construed as limited thereto.

## Claims

1. A wide-range vibration-free high-speed linear reciprocating scanning device for transferring a scanning head, comprising:
a first bevel gear rotated by a driving motor;
a crank coupled to the first bevel gear and configured to convert a rotational motion of the first bevel gear into a linear motion in association with a first link and a second link;
the first link and the second link connected to the crank, and linearly moved in response to the linear motion converted by the crank; and
a first slider and a second slider connected to the first link and the second link, respectively, and linearly moved in response to linear motions of the first link and the second link,
wherein the scanning head is connected to the first slider, and is linearly moved in response to a linear motion of the first slider.

2. The wide-range vibration-free high-speed linear reciprocating scanning device of claim 1, wherein
the crank includes:
a central shaft coupled to the first bevel gear;
two first connecting arms connected to opposite sides of the central shaft;
two first shafts connected to the two first connecting arms, respectively, and to which the first link is coupled to be rotatable;
two second connecting arms connected to the two first shafts, respectively; and
two second shafts connected to the two second connecting arms, respectively, and to which the second link is coupled to be rotatable.

3. The wide-range vibration-free high-speed linear reciprocating scanning device of claim 2, wherein
the central shaft is connected to a center of the first connecting arm,
wherein the second connecting arm is disposed to be symmetrical to the first connecting arm,
wherein a rotation center of the first connecting arm is a center of the first connecting arm, and
wherein a rotation center of the second connecting arm is a center of the second connecting arm.

4. The wide-range vibration-free high-speed linear reciprocating scanning device of claim 3, wherein
a length of section L from a rotation center to one end of each of the first connecting arm and the second connecting arm is the same as a length of section R from the rotation center to an opposite end of each of the first connecting arm and the second connecting arm.

5. The wide-range vibration-free high-speed linear reciprocating scanning device of claim 1, further comprising:
a first linear stage and a second linear stage, to which the first slider and the second slider are coupled to be linearly moved, respectively.

6. The wide-range vibration-free high-speed linear reciprocating scanning device of claim 5, further comprising:
a counter mass coupled to the second slider to be separable.
